# EUROPEAN PATENT SPECIFICATION

(11) **EP 2 224 889 B1**
(45) Date of publication and mention of the grant of the patent: **29.02.2012**
(21) Application number: 09785278.4
(22) Date of filing: 07.07.2009
(51) Int. Cl.: A61F 13/15

(54) **ABSORBENT PAD**
SAUGFÄHIGE UNTERLAGE
TAMPON ABSORBANT

(30) Priority: 07.07.2008 GB 0812363; 09.07.2008 GB 0812498; 18.08.2008 GB 0814969; 01.05.2009 GB 0907560
(43) Date of publication of application: 08.09.2010
(73) Proprietor: DRY LIKE ME LIMITED, Worcestershire WR4 9FA (GB)
(72) Inventor: HOUGH, Judith, Alcester, Warwickshire B49 6JW (GB); TITTERTON, Diane, Worcester Worcestershire WR7 4BA (GB)
(74) Representative: Johnson, Yvonne Catherine
(86) International application number: PCT/GB2009/050798
(87) International publication number: WO 2010/004322

(56) References cited:
- WO-A-01/49232
- WO-A-99/25282
- WO-A-02/087484
- WO-A-03/002698
- US-A- 5 713 886
- US-B1- 7 104 976

## Description

The present invention relates to an absorbent pad for use by children, in particular an absorbent pad for use by children with encopresis, enuresis, or other related problems.

Encopresis involves involuntary faecal leakage in children who have usually already been toilet trained, usually children aged from 4 to 14 but sometimes as old as 16. Encopresis does not involve full bowel evacuation but rather leakage of liquid or semi liquid faeces.

Encopresis may be caused by constipation, by reflexive withholding of stool, or by various physiological, psychological, or neurological disorders. For example, if a child experiences painful bowel movements the child may subsequently try to hold back bowel movement in order to avoid repetition of the painful experience. This results in build up of hardened stool, which continues to harden and build up in the colon and may even stretch the colon to the point where the normal sensations associated with impending bowel movements do not occur. Eventually, softer stool from higher up the colon leaks around the blockage and cannot be withheld, resulting in soiling.

Children with encopresis will therefore often soil their underpants with faecal matter and this can be as frequent as ten to twenty times a day.

Enuresis can also occur in children, and in this regard particular reference is made to leakages of urine during the day, as compared to nocturnal enuresis (bedwetting). These minor leakages of urine also need to be addressed for children who have been toilet trained.

The full extended colon that occurs in encopresis can put pressure on the bladder causing some degree of enuresis. Additionally, the psychological issues that accompany encopresis, in terms of a fear or nervousness of going to the toilet, can result in enuresis.

Although nappies are clearly designed to handle urine and faecal deposits, it is undesirable to place a child back into nappies once toilet training has been completed.

Further, although there are many absorbent products on the markets for lining underpants, these are predominantly designed for adult females, e.g. for menstruation, for feminine daily freshness and for female stress incontinence. These products are therefore both the wrong size and shape for children with encopresis. Additionally, for boys with encopresis there are design considerations, as such products will inevitably be made to look and smell feminine. WO 02/087484 describes an absorbent product, such as a sanitary towel, comprising a stiffening element which is intended to contribute to the three-dimensional shape of the product during its use. The stiffening element has a width at the transition between the crotch portion and the front portion on the sanitary towel which is adapted to the distance between the muscle tendons of the wearer on both sides of the crotch of the wearer in the groin of the latter and which is of the order of 15*-45 mm.

Adult incontinence pads exist but these are thick and bulky due to being designed for capturing an entire deposit from an adult. They are of course also sized for placing in adult underpants. Therefore to place these large products in a child's underpants is not dissimilar to placing the child back in nappies in terms of how the child feels and his comfort. Most adult incontinence products are also focussed on urinary incontinence and therefore are not shaped to capture faecal deposits.

Consideration must also be given to the ease of use of a product, as many children with encopresis would desire the independence of being able to address any leakages themselves in the bathroom rather than needing assistance. Equally, for younger children the parent or carer might deal with the incident but could be in a rush or might have limited dexterity due to needing to hold the child or other items at the same time. It is also important to ensure that the process of using the product is as simple and quick as possible so that any embarrassment or shame felt by the child in relation to the encopresis is not exacerbated.

There is therefore a need for an absorbent pad suitable for use by children with the above described problems.

The present invention provides an absorbent pad suitable for use by a child with encopresis and/or enuresis, the pad being designed for location inside the underpants of the child, the pad having an upper surface that presents an absorbent material and a water impervious lower surface, the pad comprising: an absorbent first section comprising absorbent material, the first section being sized for positioning in the rear gusset and back area of the child's underpants, such that it is located adjacent to the child's anal region when the child is wearing the underpants, the absorbent first section having an edge and being provided with a mesh cover; an absorbent second section, separate from or extending directly or indirectly from the first section, this second section being sized for positioning in the crotch area of the child's underpants, such that it is located adjacent to the child's urethral region when the child is wearing the underpants, wherein the second section is elongate and has a smaller area than the first section; a securing portion on the lower surface of the pad, for releasably securing the absorbent sections to the inside of the child's underpants; and one or more tabs that can be gripped by the child, located at the edge of the absorbent first section, wherein the tabs are provided with releasable securing means, such that the tabs can be folded over the edge of the underpants when the pad is located in the underpants, with the securing means contacting the outside of the underpants and releasably securing thereto.

The product of the present invention is advantageous in that the tabs can be gripped by the child to remove the pad from the underpants when the pad has been soiled and is to be replaced. This ensures that the child can easily remove the pad from the underpants by tugging the tab, rather than needing to be dextrous in finding the edge of the pad. The tab system used in the present invention also gives greater leverage, thus requiring less force to remove the pad as compared to pulling at the edges directly.

The location of the tabs at the edge of the absorbent first section is significant because this section is larger than the absorbent second section and therefore will require more effort to release. Faecal soiling in this section may also make the first section heavier than the elongate second section.

Further, this section may contain faecal soiling not only centrally in this section but also towards the edges. The person removing the pad when it is soiled, whether this is the child or the parent/carer, will wish to avoid touching the faecal soiling for hygiene reasons. Without the provision of tabs, the person removing the pad might find it difficult to hold the absorbent section whilst avoiding contact with the faecal matter, e.g. a parent/carer may be in a rush or may have their hands full, making it difficult to have the necessary dexterity. The tabs ensure that it is easy to avoid touching the faecal matter when removing the pad.

The provision of securing means on the tabs provided at the edge of the absorbent first section also provides specific benefits relevant to the problems of child encopresis. In particular, children's underpants often do not fit snugly, and they will move about during the day, especially as children are generally more active than adults. Therefore it is important that the absorbent section located in the anal area is firmly secured, to avoid smearing of faecal matter over the child's bottom and/or soiling of faecal matter on the underpants in areas that become uncovered due to movement of the pad. The tab securing means which contact the outside of the underpants and secure the pad to the underpants in the rear gusset/back area of the underpants are therefore particularly beneficial. A child with encopresis will in particular need the security of knowing that the pad is securely located in position to receive any faecal leakage and will not shift position, due to the psychological issues and nervousness commonly associated with encopresis.

The tab securing means may involve mechanical securing devices or chemical adhesives. It may, for example, involve the use of high friction material. The material may, for example, have a kinetic coefficient of friction of 0.3 or higher, 0.4 or higher, 0.5 or higher, 0.6 or higher, or 0.7 or higher. A kinetic coefficient of friction may be measured according to the ASTM D 1894 protocol.

High friction properties will grip the pad to the fibres of the underpants and therefore the tab securing means provided with the high friction material can simply be folded over the edge of the underpants and pressed onto the underpants to secure the pad to the outside of the underpants.

In one embodiment, the securing device may comprise a hook type securing device or hook and loop type securing device, such as that available under the Velcro^{®} brand. In particular, the use of a hook type securing device or hook and loop type securing device with small hooks/ loops (e.g. hooks/loops of the order of tens to hundreds of micrometres in width and height; such as from 10 to 100 micrometres in both width and height, or from 10 to 50 micrometres, in both width and height) may be contemplated, such as micro Velcro^{®}.

The benefit of such a securing device is that the hooks of such a device will attach directly to the fibres of the underpants. Therefore the tabs can simply be pressed onto the underpants in the gusset/back area to secure them to the outside of the underpants.

Therefore, as the reader will appreciate, when a hook and loop type securing device such as a Velcro^{®} securing device is used, it is only the hook sections of such a device that are required to be used in the present invention, as the fabric of the underpants provides loop sections to which the hooks can attach.

In another embodiment, the tab securing means may comprise a flocked material. As the reader will appreciate, flocked material has many small fibre 'flocks' deposited on its surface. In particular, flocked material may have short monofilament fibres on its surface. The 'flock' fibres are upstanding on the surface, which gives good slip-or-grip friction properties.

The 'flock' fibres may comprise natural or synthetic fibres, for example the fibres may be nylon, rayon or polyester. The fibres may have a diameter of, for example, from 1x10⁻⁴ m to 1x10⁻⁶m, in particular the order of about 10⁻⁵ m, e.g. from 0.5 x 10⁻⁵m to 5 x 10⁻⁵m. The fibres may have a length of, for example, from 0.1 to 10mm, preferably from 0.25 to 5mm. The fibres may be provided at a fibre density of, for example, 10 to 500 fibres per mm², e.g. from 50 to 400 fibres per mm², such as from 80 to 300 fibres per mm². The material on which the 'flock' fibres are provided may in one embodiment be a textile material but may also be paper or plastic.

Flocked material may be produced by the application of a high-voltage electric field to cause the application of the fibres to a surface of the material that has been coated with adhesive.

The flocked material may, for example, be of the type used in Fuzzy Felt^{®}. Clearly, it is preferred that the material on which the 'flock' fibres are provided is, however, flexible rather than stiff.

The benefit of such a tab securing device is that the 'flock' fibres of the material will directly secure the pad to the fibres of the underpants, due to the slip-or-grip friction properties of the flocked material. Therefore the tabs can simply be pressed onto the underpants in the gusset/back area to secure them to the outside of the underpants.

There may be any suitable number of tabs provided at the edge of the absorbent first section. Preferably, there are two or more such tabs, such as three or more, or four or more. In a preferred embodiment there are at least two such tabs and these two tabs are located such that one is on each side of the absorbent first section; most preferably they are symmetrically located on either side of the absorbent first section.

The provision of two or more such tabs, with at least one on each side, is particularly beneficial as once the pad has been soiled the pad can be folded up from end to end and the tabs from each side can then be moved together and secured to each other, to hold the pad in a folded configuration, thus sealing the faecal matter within.

Additionally, the provision of at least two such tabs, with at least one on each side, is beneficial as before the pad is used it can be folded up from end to end and the tabs from each side can then be moved together and secured to each other, to hold the pad in a folded configuration, so as to provide a smaller and more discreet product. This therefore ensures that a child is happier to carry around spare pads with them, for use as required.

Furthermore, the use of at least two tabs, one on each side, permits the absorbent first section to be lifted from the underpants once it has been soiled in a balanced way, therefore reducing the risk of any faecal matter being tipped off the pad.

The tabs may, in one embodiment, be impregnated with an antibacterial and/or antimicrobial agent. This will assist with avoiding infection from handling the pad.

The tabs may be directly attached to the absorbent first section or may be indirectly attached, e.g. via bridges. In one embodiment, bridges are used to connect the tabs to the absorbent first section and the bridges are made from extendable or elasticated material. This is advantageous because the tabs can then be moved around with a degree of freedom with respect to the absorbent section, permitting an amount of stretching to occur when using the tabs to seal up the pad in a secure folded configuration, as well as permitting easy location of the tabs on the outside of the underpants.

In one embodiment, the pad has a substantially flat upper surface that presents an absorbent material and a water impervious lower surface, which in use contacts the inside of the underpants, and the absorbent second section extends directly or indirectly from the first section.

Preferably, the absorbent first section and the absorbent second section both have substantially flat upper surfaces.

In one embodiment, the upper surface of the pad that presents an absorbent material is substantially flat. Thus both the absorbent first section and the absorbent second section, and any areas linking these sections, have substantially flat upper surfaces. The pad may optionally be provided with outer barrier walls that extend around the outer edge of some or all of this substantially flat upper surface. Such outer barrier walls can help to avoid leakage when there has been soiling.

It can be advantageous that the pad is substantially flat, rather than having ridges or protrusions from its upper or lower surfaces, as this will increase the comfort for the child and therefore make the child more relaxed about using the product.

In another embodiment, both the absorbent first section and the absorbent second section have substantially flat upper surfaces but any other areas forming part of the upper surface of the pad do not necessarily form a substantially flat upper surface with these two sections. For example, there may optionally be outer barrier walls that extend around the outer edge of some or all of the absorbent first section and/or the outer edge of some or all of the absorbent second section.

Preferably, any barrier wall that is present between the substantially flat absorbent first section and the substantially flat absorbent second section is relatively low in height, so as to maintain comfort for the child using the product. For example, such a barrier wall may have a height of 10mm or less, preferably 5mm or less, such as from 1mm to 5mm. The use of a relatively low barrier wall will increase the comfort for the child and therefore make the child more relaxed about using the product.

The barrier walls that can optionally be used in the present invention are described in more detail below.

In a preferred embodiment, the pad uses a mechanical securing device in the securing portion for securing the pad to the underpants, rather than chemical adhesives. This is beneficial in that a child need not be concerned if the pad is positioned incorrectly, because releasing and repositioning it will not reduce the strength of the bond between the pad and the underpants. This also avoids the need for peeling off a release paper from adhesive before use, which is fiddly for a child or for a parent/carer with their hands full.

Additionally, it avoids the creation of a piece of rubbish (the release paper) that needs to be disposed of.

The securing portion may comprise a high friction material. This may, for example, be a soft high friction material, such as felt, baize or flocked material. It may also be a hook and loop material such as that available under the Velcro^{®} brand.

The material may, for example, have a kinetic coefficient of friction of 0.3 or higher, 0.4 or higher, 0.5 or higher, 0.6 or higher, or 0.7 or higher. A kinetic coefficient of friction may be measured according to the ASTM D 1894 protocol.

High friction properties will grip the pad to the fibres of the underpants and therefore the parts of the pad provided with the high friction material can simply be pressed onto the underpants to secure the pad to the inside of the underpants.

In particular, in one embodiment the securing portion may comprise a hook type securing device or a hook and loop type securing device, such as that available under the Velcro^{®} brand.

In particular, the use of a hook type securing device or hook and loop type securing device with small hooks/ loops (e.g. hooks/loops of the order of tens to hundreds of micrometres in width and height; such as from 10 to 100 micrometres in both width and height, or from 10 to 50 micrometres, in both width and height) may be contemplated, such as micro Velcro^{®}.

The benefit of such a securing device is that the hooks of such a device will attach directly to the fibres of the underpants. Therefore the parts of the pad provided with the hooks can simply be pressed onto the underpants to secure the pad to the inside of the underpants.

Therefore, as the reader will appreciate, when a hook and loop type securing device such as a Velcro^{®} securing device is used, it is only the hook sections of such a device that are required to be used in the present invention, as the fabric of the underpants provides loop sections to which the hooks can attach.

In another embodiment, the securing portion may comprise a flocked material. As the reader will appreciate, flocked material has many small fibre 'flocks' deposited on its surface. In particular, flocked material may have short monofilament fibres on its surface. The 'flock' fibres are upstanding on the surface, which gives good slip-or-grip friction properties.

The 'flock' fibres may comprise natural or synthetic fibres, for example the fibres may be nylon, rayon or polyester. The fibres may have a diameter of, for example, from 1x10⁻⁴m to 1x10⁻⁶m, in particular the order of about 10⁻⁵ m, e.g. from 0.5 x 10⁻⁵m to 5 x 10⁻⁵m. The fibres may have a length of, for example, from 0.1 to 10mm, preferably from 0.25 to 5mm. The fibres may be provided at a fibre density of, for example, 10 to 500 fibres per mm², e.g. from 50 to 400 fibres per mm², such as from 80 to 300 fibres per mm². The material on which the 'flock' fibres are provided may in one embodiment be a textile material but may also be paper or plastic.

Flocked material may be produced by the application of a high-voltage electric field to cause the application of the fibres to a surface of the material that has been coated with adhesive.

The flocked material may, for example, be of the type used in Fuzzy Felt^{®}. Clearly, it is preferred that the material on which the 'flock' fibres are provided is, however, flexible rather than stiff.

The benefit of such a securing device is that the 'flock' fibres of the material will directly secure the pad to the fibres of the underpants, due to the slip-or-grip friction properties of the flocked material. Therefore the parts of the pad provided with the flocked material can simply be pressed onto the underpants to secure the pad to the inside of the underpants.

In one embodiment, the securing portion extends along most or all of the length of the pad. Preferably, it extends along 60% or more, such as 70% or more, 80% or more, preferably 90% or more, of the length of the pad.

In one embodiment, the securing portion extends over most or all of the area of the lower surface of the pad. Preferably, it extends over 60% or more, such as 70% or more, 80% or more, preferably 90% or more, of the lower surface of the pad.

The use of the securing portion over a large part of the lower surface of the pad increases the ease of secure attachment of the pad. In particular it is important that the pad can be secured easily to avoid causing distress or anxiety to the child or parent/carer.

The water impervious lower surface, on which the securing portion is provided, in use may contact the inside of the underpants. Therefore the securing portion can secure the pad to the underpants. Clearly it is only the securing portion of the water impervious lower surface that needs to contact the inside of the underpants, although some or all of the rest of the lower surface may also contact the inside of the underpants.

The absorbent first section may be any suitable shape. It may be that the first absorbent shape has substantially the same width and length. Alternatively, it may be that the width of the first absorbent shape is greater than its length. In one embodiment, the absorbent first section may be a substantially triangular shape (preferably a substantially equilateral triangle), or a substantially quadrilateral shape (preferably substantially square), or a substantially round shape (preferably substantially circular or substantially oval).

In an embodiment where the absorbent first section is a substantially triangular shape, it is preferred that the point of the triangle is at the part of this section that is closest to/adjacent the second section, with the broad end of the triangle being located furthest away from the second section.

The absorbent first section may optionally be provided with one or more extension areas extending outwardly therefrom. For example, one or more semi circular shaped extension areas may extend from the absorbent first section. In one such embodiment the absorbent first section may be a substantially equilateral triangle, or a substantially square shape, or a substantially circular shape or a substantially oval shape, and may be provided with one or more semi circular shaped extension areas around its circumference, for example two or more, three or more or four or more such extension areas.

The extension areas may be made from the same material as the absorbent first section or may, optionally, be made from different material. In one embodiment the extension areas may be made from a thinner material than the absorbent first section and/or a less absorbent material than the absorbent first section.

The absorbent first section suitably has a maximum width that is greater than or equal to the maximum width of the absorbent second section. The absorbent first section suitably has a minimum width that is greater than or equal to the minimum width of the absorbent second section.

In one embodiment, the absorbent first section has a minimum width that is greater than or equal to the maximum width of the absorbent second section.

Generally, the absorbent second section has a width suited to fit the width of the crotch section of the child's underpants. The absorbent first section, however, is preferably wider than this, to ensure that all faecal matter that is expelled falls onto this section. Preferably, the maximum width of the absorbent first section is at least 1.5 times, e.g. 2 times, the minimum width of second section.

The area of the absorbent first section may suitably be at least twice that of the absorbent second section, such as three times or more or four times or more.

The second section may be any suitable shape that will fit the crotch section of the child's underpants. It may have a substantially constant width, for example, it may be substantially rectangular, or it may be a tapered shape. In one embodiment it is an isosceles trapezoid shape. In an embodiment where the absorbent second section is a tapered shape, it is preferred that the broader end is at the part of this section that is closest to/adjacent the first section, with the narrower end being located furthest away from the first section.

The absorbent first section and second absorbent section may be directly adjacent or they may be joined by a bridging section. Alternatively, there may be a gap of non absorbent material between the absorbent first section and second absorbent section.

Preferably the pad is made from materials such that it is flushable. In one embodiment the pad is made from materials that can all dissolve or disintegrate in water over a period of time. These are known in the art.

The second section is an absorbent section; this will permit minor urine leaks to be absorbed by this section. As noted above, children with encopresis will often also have a degree of enuresis. The second section may be made from the same absorbent material as the first section, or may be made from a different absorbent material.

The absorbent sections of the pad may be formed from any absorbent material or combinations of absorbent material. These materials are well known in the fields of incontinence pads and nappies. For example, cotton wool or similar absorbent materials may be considered.

The first section and/or the second section may additionally be provided with absorbent crystals that can absorb fluids. For example, gel crystals may be provided. The inclusion of such crystals within the pad is beneficial as it will act to wick fluids away from the surface of the pad that is in contact with the child.

In one embodiment, there is a barrier provided between the first section and the second section, which acts to prevent faecal matter from travelling from the first section to the second section. This is particularly relevant when the pad is for a girl, as it will avoid any faecal matter potentially causing an infection in the vaginal area. The barrier may, for example, be stitched or adhered to the region between the first section and second section.

In another embodiment, there is a gap provided between the first section and the second section, which acts to prevent faecal matter from travelling from the first section to the second section. Again, this is particularly relevant when the pad is for a girl, as it will avoid any faecal matter potentially causing an infection in the vaginal area.

In an embodiment where a gap is provided, this gap should ideally be small, so as to reduce the area where there is not absorbent material. For example, at the closest point between the first section and the second section the gap may have a maximum width of 5mm or less, preferably 4mm or less, such as 3mm or less, or 2mm or less, e.g. 1mm or less.

The absorbent first section is provided with a mesh cover. The mesh may stick to faecal matter, thus ensuring that it sticks to the pad and comes away from the child's body. Alternatively, the faecal matter may fall through the holes in the mesh, and become trapped beneath the mesh cover; again ensuring the faeces is away from the child's body. The mesh cover may, for example, have a honeycomb style weave. In one embodiment, the mesh cover has different sized holes, to help capture and trap faecal matter.

The absorbent first section and/or the absorbent second section may optionally be provided with outer barrier walls. These walls can act to prevent overflow and leaking when soiling has occurred. These walls, when present, may extend around some or all of the outer edge of the absorbent section in question.

For example, an outer barrier wall may extend around most or all of the outer edge of the first absorbent section. Preferably, it extends over 60% or more, such as 70% or more, 80% or more, preferably 90% or more, of the outer edge of the first absorbent section.

For example, an outer barrier wall may extend around most or all of the outer edge of the second absorbent section. Preferably, it extends over 60% or more, such as 70% or more, 80% or more, preferably 90% or more, of the outer edge of the second absorbent section.

In one embodiment, the outer barrier walls extend around some of the outer edge of the first absorbent section and some of the outer edge of the second absorbent section, such that the outer barrier walls extend only around the outer edge of the upper surface of the pad.

In another embodiment, the outer barrier walls extend around all of the outer edge of the first absorbent section and all of the outer edge of the second absorbent section.

In another embodiment, the outer barrier walls extend around only the outer edge of the first absorbent section.

The reader will appreciate that the barrier walls may be located at or near the outer edge of the absorbent section in question. Whilst in one embodiment the walls are positioned at the outer edge, in another embodiment they may be positioned slightly inside the outer edge, for example within 1cm or less of the outer edge, such as 0.5cm or less, e.g. 0.25cm or less.

The barrier walls may be made from any material. In one embodiment, the barrier walls are made from absorbent material, such as the absorbent material discussed above. In another embodiment the barrier walls are made of waterproof material or have waterproof material on their outermost surface.

It may be that the barrier walls are produced by being a continuation of the absorbent section - for example the barrier walls may be formed by turning up the edges of the absorbent material to form an upwardly extending wall, or may be formed by providing a thicker section of absorbent material at the edges of the absorbent section.

The barrier walls may, for example, have a height of 10mm or less, e.g. from 1mm to 10mm, such as from 2mm to 5mm. The barrier walls may, for example, have a thickness of from 1mm to 10mm, such as from 2mm to 5mm.

The first section and/or the second section may be impregnated with soap free, pH balanced, cleansing agent. In particular, soap free cleansing foam, e.g. Vernacare Senset skin cleansing foam, which can loosen and lift soiling from the skin, may be used. Cleansing agents that have high wetting out properties are particularly beneficial in this regard.

Ideally, the agent may provide a high water repellent barrier upon contact with the skin, thus preventing dermatitis or other skin inflammation developing from damp skin, and protecting the skin from the acid effects of urine.

The pad has a water impervious lower surface. This may be achieved by providing a waterproof backing to the pad, to prevent fluids from seeping through to the underpants of the child.

The pad may include an odour neutraliser and/or a fragrance.

The pad is preferably from 10cm to 25cm in length, more preferably from 15 to 20cm in length.

The pad suitably has a maximum width, which is present in the first absorbent section, of from 5 to 15cm, preferably from 7 to 12 cm.

The pad suitably has a minimum width, which is present in the second absorbent section, of from 1cm to 10cm, preferably from 1.5cm to 5cm.

The pad suitably has a depth of 1.5cm or less, such as from 0.1cm to 1cm.

In a second aspect, the invention provides a kit comprising a pad in accordance with the first aspect in combination with a pouch sized to contain the pad.

The pouch may, for example, be a plastic pouch or a fabric pouch. The pouch may preferably be made of biodegradable material, such as biodegradable plastic.

The pouch is preferably sealable, and most preferably re-sealable. This is advantageous in that the unused pad can be provided in the pouch and sealed therein until it is required for use. Once the pad has been soiled, it can be replaced into the pouch, and the pouch can be resealed, to secure the soiled product. This enables the soiled product to be kept in a hygienic fashion until a suitable disposal point is reached.

The pouch may, for example, have a top opening that is covered by a flap that can be opened and re-sealably closed.

The invention will now be further described, by means of example only, with reference to the drawings in which:
**Figure 1** is a plan view from above of a pad in accordance with the invention;
**Figure 2** is a plan view from below of the pad of Figure 1;
**Figure 3** is a cross sectional view through the pad of Figure 1;
**Figure 4** is a view from above of the pad of Figure 1 in a partially folded configuration;
**Figure 5** is a view from above of the pad of Figure 1 in a fully folded configuration;
**Figure 6** is a plan view from above illustrating an alternative configuration for the absorbent sections of a pad in accordance with the invention;
**Figure 7a and 7b** are plan views from above illustrating an optional barrier wall feature for pads in accordance with the invention; and
**Figure 8** is a plan view from below illustrating an alternative configuration for the securing portions of a pad in accordance with the invention.

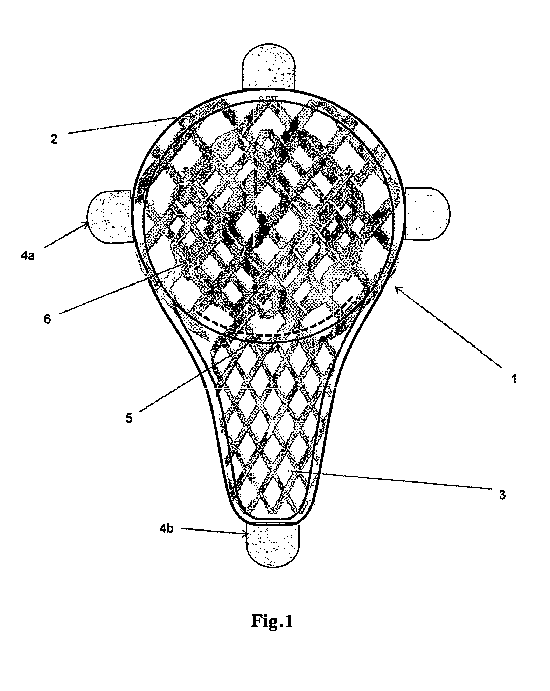

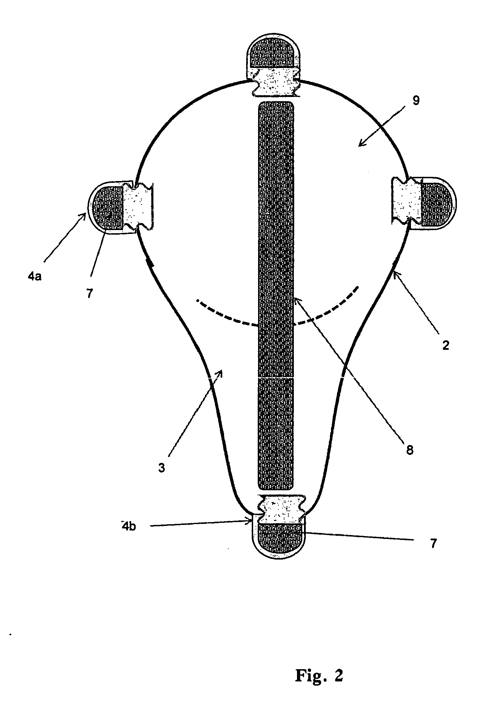

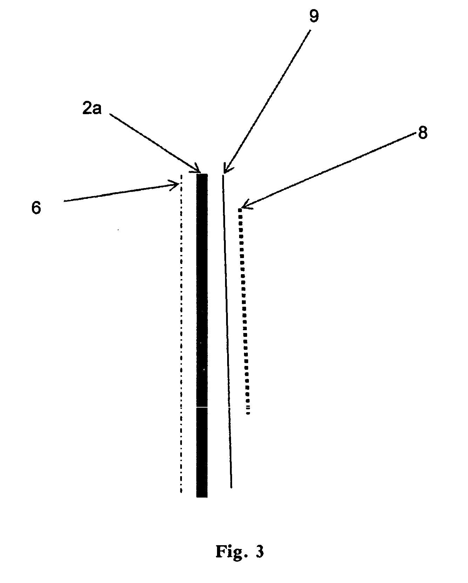

The pad shown in Figures 1 to 3 is an absorbent pad 1 suitable for use by a child with encopresis and/or enuresis. The pad is for location inside the underpants of the child.

The pad has a substantially flat upper surface that presents an absorbent material and a water impervious lower surface, which in use contacts the inside of the underpants. The water impervious lower surface comprises a waterproof backing 9.

The pad has a substantially flat absorbent first section 2 comprising absorbent material 2a. This first section is sized for positioning in the rear gusset and back area of the child's underpants, such that it is located adjacent to the child's anal region when the child is wearing the underpants.

The pad also has a substantially flat absorbent second section 3, which extends directly from the first section 2. This second section is sized for positioning in the crotch area of the child's underpants, such that it is located adjacent to the child's urethral region when the child is wearing the underpants.

It can be seen that the first section is substantially circular, whilst the second section is a tapered shape that narrows from the first section. The second section has a smaller area than the first section.

The pad also includes a securing portion 8 on the lower surface of the pad, for releasably securing the absorbent sections 2, 3 to the inside of the child's underpants. This is a strip of micro Velcro^{®} that runs substantially centrally along the length of the pad. The strip is the hook providing strip, rather than the loop providing strip, of the Velcro^{®}. The hooks of this micro Velcro^{®} strip will attach directly to the fibres of the underpants. Therefore the pad can simply be pressed onto the underpants to secure the pad to the inside of the underpants. Equally, the securing portion 8 may be any other high friction material, such as flocked material, that will grip to the underpants to secure the pad.

The pad also includes two tabs 4a that can be gripped by the child. These two tabs are symmetrically located on either side of the absorbent first section 2.

The tabs 4a are provided with releasable securing means 7, which is a section of micro Velcro^{®}. The strip is the hook providing strip, rather than the loop providing strip, of the Velcro^{®}. The hooks of this micro Velcro^{®} strip will attach directly to the fibres of the underpants. This means that the tabs 4a can be folded over the edge of the underpants when the pad 1 is located in the underpants, with the micro Velcro^{®} securing means contacting the outside of the underpants and releasably securing thereto. These tabs can also be used to assist with securing the pad in a folded configuration. Equally, the securing means 7 may be any other high friction material, such as flocked material, that will grip to the underpants to secure the pad.

The pad 1 is also provided with additional tabs 4b, one at each end. These are substantially in line with the securing strip of micro Velcro^{®} 8 that runs along the length of the pad. These additional tabs 4b are provided with releasable securing means 7, which is a section of micro Velcro^{®}. This means that the tabs 4b can also be used to help secure the pad in position in the child's underpants. These tabs can also be used to assist with securing the pad in a folded configuration. The tabs 4a, 4b are indirectly attached to the pad 1 via bridges (not shown). The bridges are made from elasticated material. This provides flexibility in the movement of the tabs.

There is a stitched or bonded barrier 5 provided between the absorbent sections of the pad 2, 3, which barrier acts to prevent faecal matter from travelling from the first section to the second section.

The absorbent sections of the pad 2, 3 may be formed from any absorbent material 2a, for example, cotton wool or similar absorbent materials. The absorbent sections of the pad 2, 3 may be impregnated with soap free, pH balanced, cleansing foam, e.g. Vernacare Senset skin cleansing foam, which can loosen and lift soiling from the skin.

The absorbent first section 2 is provided with a mesh cover 6 with a honeycomb style weave. The mesh cover has different sized holes, to help capture and trap faecal matter.

The absorbent sections of the pad 2, 3 may optionally be provided with absorbent gel crystals (not shown) that can absorb fluids. The absorbent sections of the pad 2, 3 may include an odour neutraliser and/or a fragrance.

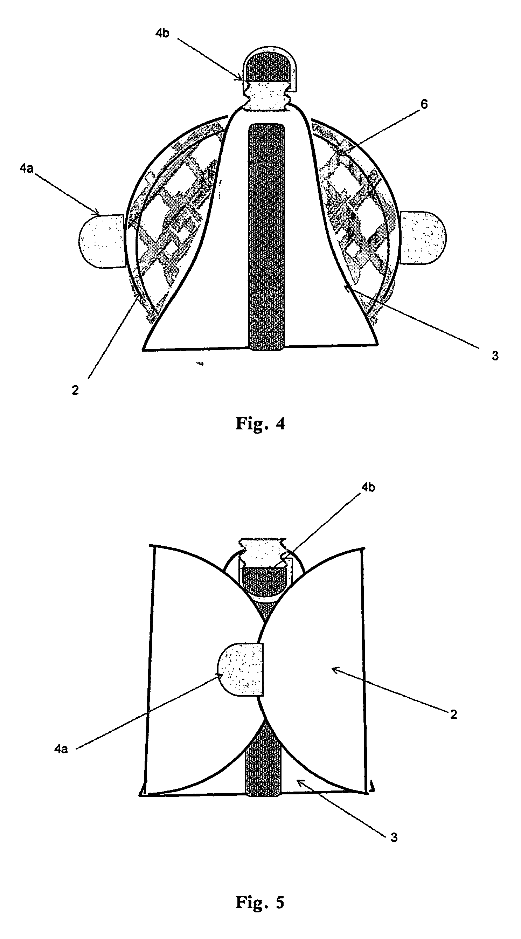

Figures 4 and 5 show how the pad can be folded up, both for storage before use and to contain faecal matter after use.

In Figure 4 it can be seen that the second section 3 can be folded over the first section 2, such that the waterproof backing is on the outside and the absorbent material 2a and mesh 6 are on the inside. This will bring the tabs 4b from each end of the pad over each other. The tabs 4b at each end of the pad can then be secured together using the micro Velcro^{®} securing means 7 on the tabs, to keep the pad in this folded configuration.

In Figure 5 it can be seen that the two edge tabs 4a can then be used to bring the edges of the first section 2 together, such that the waterproof backing is on the outside, with the tabs 4a then being secured together centrally. This ensures that any waste caught in the absorbent material 2a and mesh 6 is secured inside a folded packet. The micro Velcro^{®} securing means 7 on the tabs 4a secure the tabs together, keeping the pad in the folded packet configuration.

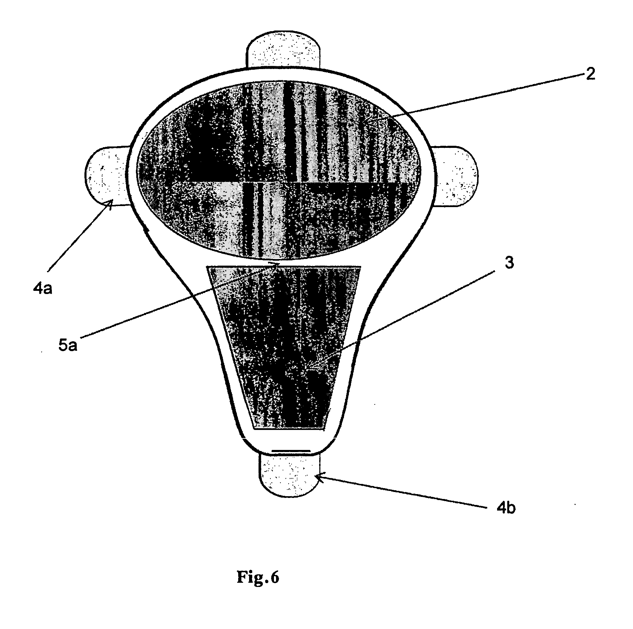

Figure 6 shows a variation of the pad where there is a gap 5a provided between the first absorbent section 2 and the second absorbent section 3, which acts to prevent faecal matter from travelling from the first section to the second section.

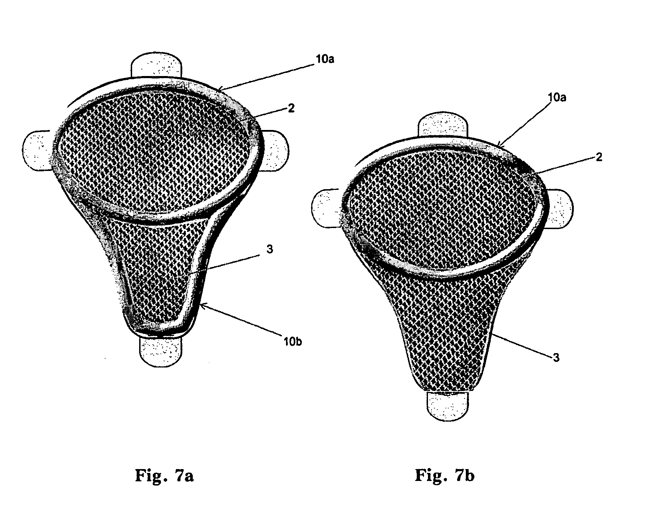

Figures 7a and 7b show variations of the pad where the substantially flat absorbent first section 2 and/or the substantially flat absorbent second section 3 are provided with outer barrier walls 10a, 10b. Figure 7a shows the absorbent first section 2 provided with outer barrier walls 10a and the absorbent second section 3 provided with outer barrier walls 10b. Figure 7b shows an embodiment where only the absorbent first section 2 is provided with outer barrier walls 10a.

The barrier walls act to prevent overflow and leaking when soiling has occurred.

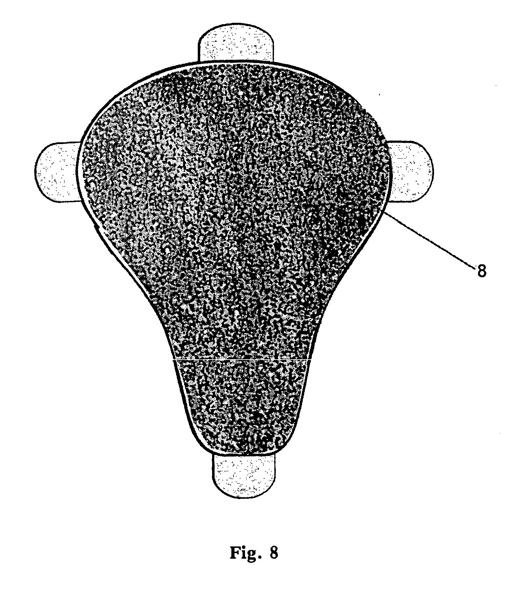

Figure 8 shows a variation of the pad, where the securing portion 8 extends over the entire lower surface of the pad.

## Claims

1. An absorbent pad (1) suitable for use by a child with encopresis and/or enuresis, the pad being designed for location inside the underpants of the child, the pad having an upper surface that presents an absorbent material and a water impervious lower surface, the pad comprising:
an absorbent first section (2) comprising absorbent material (2a), the first section being sized for positioning in the rear gusset and back area of the child's underpants, such that it is located adjacent to the child's anal region when the child is wearing the underpants, the absorbent first section having an edge;
an absorbent second section (3), separate from or extending directly or indirectly from the first section, this second section being sized for positioning in the crotch area of the child's underpants, such that it is located adjacent to the child's urethral region when the child is wearing the underpants, wherein the second section is elongate and has a smaller area than the first section;
a securing portion (8) on the lower surface of the pad, for releasably securing the absorbent sections to the inside of the child's underpants;
**characterised in that**
the pad further comprises one or more tabs (4a) that can be gripped by the child, located at the edge of the absorbent first section, wherein the tabs are provided with releasable securing means (7), such that the tabs can be folded over the edge of the underpants when the pad is located in the underpants, with the securing means contacting the outside of the underpants and releasably securing thereto; and
wherein the absorbent first section is provided with a mesh cover (6).

2. The pad of claim 1, wherein the tab securing means comprises a high friction material, such as a flocked material, or a hook type securing device, or hook and loop type securing device.

3. The pad of claim 1 or claim 2, wherein two or more tabs are provided at the edge of the absorbent first section and these tabs are located such that at least one tab is on each side of the absorbent first section.

4. The pad of any one of the preceding claims wherein the tabs are indirectly attached to the absorbent first section via bridges and the bridges are made from extendable or elasticated material.

5. The pad of any one of the preceding claims wherein the securing portion comprises a high friction material, such as a flocked material, or a hook type securing device, or a hook and loop type securing device.

6. The pad of any one of the preceding claims wherein the absorbent first section has a minimum width that is greater than or equal to the maximum width of the absorbent second section.

7. The pad of any one of the preceding claims wherein there is a barrier (5) or gap (5a) provided between the first section and the second section, which acts to prevent faecal matter from travelling from the first section to the second section.

8. The pad of any one of the preceding claims wherein the mesh cover has different sized holes, to help capture and trap faecal matter.

9. The pad of any one of the preceding claims wherein the first section and/or the second section is impregnated with soap free, pH balanced, cleansing agent.

10. The pad of any one of the preceding claims, wherein the absorbent first section and the absorbent second section both have substantially flat upper surfaces.

11. The pad of any one of the preceding claims, wherein the upper surface of the pad that presents an absorbent material is substantially flat.

12. The pad of any one of the preceding claims, wherein the pad is provided with outer barrier walls (10a, 10b) that extend around the outer edge of some or all of the absorbent first section and/or the outer edge of some or all of the absorbent second section.

13. A kit comprising a pad in accordance with any one of claims 1 to 12, in combination with a pouch sized to contain the pad.

14. The kit of claim 13, wherein the pouch is made of biodegradable plastic.

15. The kit of claim 13 or claim 14, wherein the pouch is sealable and re-sealable.

## Patentansprüche

1. Eine saugfähige Einlage (1), geeignet für die Verwendung durch ein Kind, das an Enkopresis und/oder Enuresis leidet, wobei die Einlage für die Anbringung in der Unterhose des Kindes vorgesehen ist und die Einlage an der Oberseite ein saugfähiges Material aufweist und an der Unterseite wasserundurchlässig ist; die Einlage beinhaltet Folgendes:
einen saugfähigen ersten Abschnitt (2), der saugfähiges Material (2a) umfasst, wobei der erste Abschnitt die entsprechende Größe hat, um im hinteren Zwickel und dem hinteren Bereich der Unterhose Kindes eingelegt zu werden, sodass er in der Nähe des Analbereichs des Kindes aufliegt, wenn das Kind die Unterhose trägt, wobei der saugfähige erste Abschnitt einen Rand aufweist;
einen saugfähigen zweiten Abschnitt (3), der vom ersten Abschnitt getrennt ist oder direkt oder indirekt vom ersten Abschnitt ausgeht, wobei dieser zweite Abschnitt die entsprechend Größe hat, um im Schritt der Unterhose des Kindes eingelegt zu werden, sodass er in der Nähe des Harnröhrenbereichs des Kindes aufliegt, wenn das Kind die Unterhose trägt, wobei der zweite Abschnitt länglich ist und eine kleinere Fläche als der erste Abschnitt aufweist:
einen Befestigungsteil (8) auf der Unterseite der Einlage, um die saugfähigen Abschnitte lösbar auf der Innenseite der Unterhose des Kindes zu fixieren;
**dadurch gekennzeichnet, dass**
die Einlage überdies eine oder mehrere Laschen (4a) umfasst, die das Kind ergreifen kann und die sich am Rand des saugfähigen ersten Abschnitts befinden, worin die Laschen mit einer lösbaren Befestigungsvorrichtung (7) versehen sind, sodass die Laschen über die Kante der Unterhose gefaltet werden können, wenn die Einlage in die Unterhose gelegt wird, wobei die Befestigungsvorrichtung mit der Außenseite der Unterhose in Berührung kommt und lösbar daran befestigt wird; und
worin der saugfähige erste Abschnitt mit einer Netzabdeckung (6) ausgestattet ist.

2. Die Einlage nach Anspruch 1, worin es sich bei der Befestigungslasche um ein reibungsbeständiges Material, z.B. geflocktes Material, oder eine Fixiervorrichtung aus Hakenhaftelementen bzw. einen Klettverschluss handelt.

3. Die Einlage nach Anspruch 1 oder Anspruch 2, worin zwei oder mehrere Laschen am Rand des saugfähigen ersten Abschnitts vorhanden sind und diese Laschen so positioniert sind, dass sich mindestens eine Lasche auf jeder Seite des saugfähigen ersten Abschnitts befindet.

4. Die Einlage nach einem der vorhergehenden Ansprüche, worin die Laschen über Stege indirekt am saugfähigen ersten Abschnitt befestigt sind und die Stege aus ausdehnbarem oder elastischem Material hergestellt sind.

5. Die Einlage nach einem der vorhergehenden Ansprüche, worin die Befestigungslasche ein reibungsbeständiges Material, z.B. geflocktes Material, oder eine Fixiervorrichtung aus Hakenhaftelementen bzw. einen Klettverschluss umfasst.

6. Die Einlage nach einem der vorhergehenden Ansprüche, worin der saugfähige erste Abschnitt eine Mindestbreite hat, die größer als oder gleich wie die Höchstbreite des saugfähigen zweiten Abschnitts ist.

7. Die Einlage nach einem der vorhergehenden Ansprüche, worin eine Barriere (5) oder ein Abstand (5a) zwischen dem ersten und dem zweiten Abschnitt vorhanden ist, was verhindert, dass fäkales Material vom ersten zum zweiten Abschnitt gelangen kann.

8. Die Einlage nach einem der vorhergehenden Ansprüche, worin die Netzabdeckung verschieden große Löcher hat, die helfen, fäkales Material abzufangen und aufzunehmen.

9. Die Einlage nach einem der vorhergehenden Ansprüche, worin der erste Abschnitt und/oder der zweite Abschnitt mit einem seifenfreien, pH-neutralen Reinigungsmittel imprägniert ist.

10. Die Einlage nach einem der vorhergehenden Ansprüche, worin sowohl der saugfähige erste Abschnitt als auch der saugfähige zweite Abschnitt eine überwiegend flache Oberseite haben.

11. Die Einlage nach einem der vorhergehenden Ansprüche, worin die Oberseite der Einlage, die ein saugfähiges Material aufweist, überwiegend flach ist.

12. Die Einlage nach einem der vorhergehenden Ansprüche, worin die Einlage mit äußeren Schutzwänden (10a, 10b) versehen ist, die sich um den äußeren Rand eines Teils oder des gesamten saugfähigen ersten Abschnitts und/oder um den äußeren Rand eines Teils oder des gesamten saugfähigen zweiten Abschnitts erstreckt.

13. Ein Set, das eine Einlage entsprechend einem der Ansprüche 1 bis 12 umfasst, in Kombination mit einem Beutel, der entsprechend geformt ist, um die Einlage aufzunehmen.

14. Das Set nach Anspruch 13, worin der Beutel aus biologisch abbaubarem Plastik hergestellt ist.

15. Das Set nach Anspruch 13 oder Anspruch 14, worin der Beutel verschließbar und wieder verschließbar ist.

## Revendications

1. Un tampon absorbant (1) adapté pour une utilisation par un enfant souffrant d'encoprésie et/ou d'énurésie, le tampon étant conçu pour un placement à l'intérieur du sous-vêtement de l'enfant, le tampon possédant une surface supérieure qui présente un matériau absorbant et une surface inférieure imperméable à l'eau, le tampon comprenant :
une première partie absorbante (2) comprenant un matériau absorbant (2a), la première partie étant dimensionnée pour un positionnement dans le gousset arrière et la zone dorsale du sous-vêtement de l'enfant, de sorte qu'elle soit placée de manière adjacente à la zone anale de l'enfant lorsque l'enfant porte le sous-vêtement, la première partie absorbante possédant une bordure,
une deuxième partie absorbante (3), séparée de ou s'étendant directement ou indirectement à partir de la première partie, cette deuxième partie étant dimensionnée pour un positionnement dans la zone d'entrejambe du sous-vêtement de l'enfant, de sorte qu'elle soit placée de manière adjacente à la zone urétrale de l'enfant lorsque l'enfant porte le sous-vêtement, où la deuxième partie est allongée et a une superficie plus petite que la première partie,
une partie de fixation (8) sur la surface inférieure du tampon, pour une fixation libérable des parties absorbantes à l'intérieur du sous-vêtement de l'enfant,
**caractérisé en ce que**
le tampon comprend en outre une ou plusieurs languettes (4a) qui peuvent être saisies par l'enfant, placées à la bordure de la première partie absorbante, où les languettes sont munies d'un moyen de fixation libérable (7), de sorte que les languettes puissent être repliées par dessus la bordure du sous-vêtement lorsque le tampon est placé dans le sous-vêtement, avec le moyen de fixation en contact avec l'extérieur du sous-vêtement et fixé de manière libérable à celui-ci, et
où la première partie absorbante est munie d'une couverture maillée (6).

2. Le tampon selon la Revendication 1, où le moyen de fixation du tampon comprend un matériau à coefficient de frottement élevé, tel qu'un matériau floqué, ou un dispositif de fixation de type à crochets, ou un dispositif de fixation de type à boucles et à crochets.

3. Le tampon selon la Revendication 1 ou 2, où deux ou plus languettes sont placées à la bordure de la première partie absorbante et ces languettes sont placées de sorte qu'au moins une languette se trouve sur chaque côté de la première partie absorbante.

4. Le tampon selon l'une quelconque des Revendications précédentes où les languettes sont fixées indirectement à la première partie absorbante par des ponts et les ponts sont fabriqués dans un matériau élastique ou extensible.

5. Le tampon selon l'une quelconque des Revendications précédentes où la partie de fixation comprend un matériau à coefficient de frottement élevé, tel qu'un matériau floqué, ou un dispositif de fixation de type à crochets, ou un dispositif de fixation de type à boucles et à crochets.

6. Le tampon selon l'une quelconque des Revendications précédentes où la première partie absorbante possède une largeur minimale qui supérieure à ou égale à la largeur maximale de la deuxième partie absorbante.

7. Le tampon selon l'une quelconque des Revendications précédentes où il existe une barrière (5) ou un espace (5a) placé entre la première partie et la deuxième partie qui agit de façon à empêcher la matière fécale de circuler de la première partie à la deuxième partie.

8. Le tampon selon l'une quelconque des Revendications précédentes où la couverture maillée possède des trous de différentes tailles destinés à aider à capturer et piéger la matière fécale.

9. Le tampon selon l'une quelconque des Revendications précédentes où la première partie et/ou la deuxième partie est imprégnée d'un agent nettoyant sans savon à pH équilibré.

10. Le tampon selon l'une quelconque des Revendications précédentes, où la première partie absorbante et la deuxième partie absorbante possèdent toutes les deux des surfaces supérieures sensiblement planes.

11. Le tampon selon l'une quelconque des Revendications précédentes, où la surface supérieure du tampon qui présente un matériau absorbant est sensiblement plane.

12. Le tampon selon l'une quelconque des Revendications précédentes, où le tampon est muni de parois barrières extérieures (10a, 10b) qui s'étendent autour de la bordure extérieure d'une partie ou de la totalité de la première partie absorbante et/ou de la bordure extérieure d'une partie ou de la totalité de la deuxième partie absorbante.

13. Un kit contenant un tampon selon l'une quelconque des Revendications 1 à 12, en combinaison avec une poche dimensionnée de façon à contenir le tampon.

14. Le kit selon la Revendication 13, où la poche est fabriquée dans un plastique biodégradable.

15. Le kit selon la Revendication 13 ou 14, où la poche est scellable et re-scellable.
